# EUROPEAN PATENT APPLICATION

(11) **EP 0 724 156 A1**
(43) Date of publication of application: **31.07.1996**
(21) Application number: 96100988.3
(22) Date of filing: 24.01.1996
(51) Int. Cl.: G01N 33/543, G01N 33/53, G01N 33/58

(54) **Kit for immunologically assaying biological substance and assay process**

(30) Priority: 26.01.1995 JP 10627/95
(71) Applicant: NIPPON PAINT CO., LTD., Osaka-shi Osaka 530 (JP)
(72) Inventor: Takahashi, Yoji, Tokyo (JP); Kabayashi, Hisaka, London NW3 3EJ (GB)
(74) Representative: Hansen, Bernd, Dr. Dipl.-Chem.

(57) **Abstract**

An immunoassay kit and an immunoassay process are provided that enables a convenient assay of a biological substance without inducing prozone phenomenon. The immunoassay kit comprises at least magnetic particles (A) immobilized with a substance that immunologically binds to the analyte substance; and non-magnetic particles (B) immobilized with the analyte substance. The immunoassay kit may further comprise magnetic particles (C) immobilized with a substance that immunologically binds to an assay interfering substance in the sample. The immunoassay process of the present invention is carried out by reacting the sample with the particles (A) and (C), and with the particles (B); collecting the magnetic particles that failed to react and that are in the form of immunocomplexes; and measuring absorbance of the non-magnetic particles remaining in the reaction solution. The analyte in the sample is quantitatively determined by referring to the calibration curve that has been prepared by using the standard samples of the analyte substance.

## Description

### BACKGROUND OF THE INVENTION

This invention relates to a process for assaying a biological substance in a sample by means of an antigen-antibody reaction utilizing magnetic and non-magnetic particles wherein a body fluid can be used for the sample with no influence of the substances that interfere with the assay. This invention also relates to a kit for such process. This process does not require any complicated procedure such as B/F (bound/free) separation by which immunocomplex formed between the antigen and the antibody is separated from the free non-reacted substances. This process also enables a reliable measurement of the analyte substance at a high sensitivity, and absorbance value of an increase in the concentration of the analyte substance is detected as an increase in the value of absorbance.

Antigens and antibodies in the body fluid samples have been measured by various immunoassays. Conventional such immunoassays include an assay of analyte substance in a body fluid such as serum, plasma, urine or the like using magnetic particles immobilized with an antigen or an antibody. Such assays are disclosed in Japanese Patent Application Laid-Open Nos. 55(1980)-156866, 61(1986)-128168, 1(1989)-193647, 3(1991)-59459, and 3(1991)-128462.

Japanese Patent Application Laid-Open No. 55(1980)-156866 discloses a process wherein an analyte substance in the sample solution is assayed by adding first magnetic particles immobilized with an antigen or an antibody to the sample solution to allow the formation of an immunocomplex between the magnetic particle and the analyte substance; adding second particles capable of binding to said immunocomplex but incapable of reacting with the free first particle that failed to form the immunocomplex to allow the binding of the second particles to the immunocomplex thereby facilitating agglutination of the immunocomplex; and selectively detecting the first and second particles that failed to agglutinate by means of their labels to measure the amount of the analyte antigen or antibody in the sample solution. There are also disclosed that the particles used may be magnetic particles, and the separation of the agglutinated immunocomplex may be carried out by centrifugation or magnetic gathering.

Japanese Patent Application Laid-Open No. 1(1989)-193647 discloses an assay process wherein magnetic particles immobilized with an antibody or an antigen and non-magnetic particles immobilized with an antibody or an antigen are reacted with an antigen or an antibody to form agglutinates; the agglutinates are separated from the reaction solution; and the non-magnetic particles remaining in the reaction solution are evaluated for their amount by measuring absorbance or scattering of light to thereby qualitatively or quantitatively determine the amount of the analyte substance.

Japanese Patent Application Laid-Open No. 3(1991)-59459 discloses an assay process wherein agglutinates are formed between the magnetic particles and the non-magnetic particles; the agglutinates are separated from the reaction solution; and the non-magnetic particles remaining in the reaction solution are evaluated for their amount by turbidimetry to thereby qualitatively or quantitatively determine the amount of the analyte substance.

Japanese Patent Application Laid-Open No. 3(1991)-128462 discloses an assay process wherein magnetic particles that reacted or failed to react with an antibody or an antigen as well as agglutinates of the magnetic particles with the non-magnetic particles are separated from the reaction solution; and the non-magnetic particles remaining in the reaction solution are evaluated for their amount by visual inspection with naked eyes or by optical measurement to quantitatively determine the amount of the analyte antibody, followed by the determination of its Ig class.

Japanese Patent Application Laid-Open No. 61(1986)-128168 discloses a process wherein an analyte substance in the sample solution is assayed by reacting insoluble magnetizable carrier (magnetic particles) immobilized with an antibody or an antigen with labeled non-magnetizable particles (non-magnetic particles); applying magnetic field to the reaction mixture to separate the magnetized particles and agglutinates containing the magnetized particles in the reaction mixture by means of magnetic gathering; and evaluating the label intensity of the labeled particles in the agglutinates to thereby determine the amount of the analyte substance in the sample solution.

For the purpose of obtaining a calibration curve in which absorbance increases with the increase in the concentration of the antigen, in the above-described assays, the assay has been carried out by reacting the magnetic particles immobilized with the antibody or the antigen with the labeled non-magnetic particles; conducting B/F separation between the immunocomplex formed between the two types of particles and the remaining non-magnetic particles that escaped the magnetic gathering and washing the separated immunocomplex; and detecting the amount of the non-magnetic particles in the immunocomplex by means of the label. Therefore, the B/F separation and the subsequent washing steps were indispensable in the above-described assay procedures.

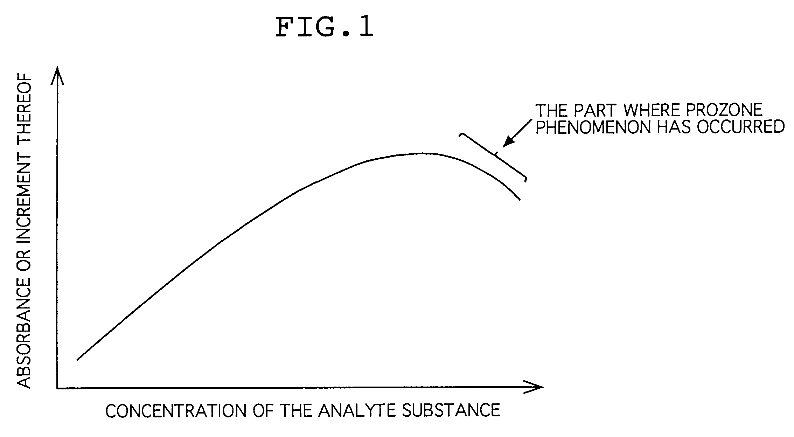

The calibration curves in the above-described assays ascend with the increase in the concentration of the analyte substance as in the case of the calibration curves generally obtained in an immunoassay or EIA. However, when an excessive amount of antigen is used in latex agglutination or turbidimetry, there were some cases wherein formation of the antigen-antibody immunocomplex failed to occur to result in the abrupt reduction on the absorbance curve, so called prozone phenomenon as shown in FIG. 1. In FIG. 1, the part of the curve exhibiting the prozone phenomenon is indicated by an arrow.

Furthermore, the sample to be assayed may contain a number of biological substances in addition to the analyte substance, and some of such biological substances may interfere with the assay results. Conventional assays as described above required such assay-interfering biological substances to be removed before the assay since presence of such substance resulted in poor assay precision, and hence unreliability of the assay.

### SUMMARY OF THE INVENTION

The present invention has been completed to obviate the situation as described above, and an object of the present invention is to provide an assay kit and an assay process that provides an ascending calibration curve where the absorbance increases with the increase in the concentration of the analyte substance with no prozone phenomenon even if washing step were omitted, and that is highly reliable showing an excellent assay precision.

According to the present invention, there is provided a kit for immunologically assaying an analyte biological substance in a sample by utilizing an antigen-antibody reaction comprising
insoluble magnetic particles immobilized with a substance that immunologically binds to the analyte substance; and
insoluble non-magnetic particles having an absorption wave length of a particular range immobilized with the analyte substance.

The immunoassay kit of the present invention may further comprise insoluble magnetic particles immobilized with a substance that immunologically binds to a substance in the sample that interferes with the assay.

Furthermore, there is provided by the present invention a process for assaying an analyte biological substance in a sample by utilizing an antigen-antibody reaction comprising the steps of
(1) mixing a dispersion of insoluble magnetic particles immobilized with a substance that immunologically binds to the analyte substance and a dispersion of insoluble non-magnetic particles having an absorption wave length of a particular range immobilized with the analyte substance with the sample in an arbitrary order to produce a mixture;
(2) applying a magnetic field to said mixture to collect complexes of said magnetic particle and said analyte substance formed in said mixture together with the magnetic particles that failed to bind immunologically; and
(3) determining concentration of said non-magnetic particles by measuring their absorbance.

In the immunoassay process of the present invention, insoluble magnetic particles immobilized with a substance that immunologically binds to a substance in the sample that interferes with the assay may be further added to said mixture; and complex of said magnetic particle and said assay interfering substance formed in said mixture may also be collected by the magnetic field.

### BRIEF DESCRIPTION OF THE DRAWINGS

FIG. 1 shows a calibration curve depicted in a typical conventional immunoassay. Occurrence of prozone phenomenon is demonstrated by the calibration curve.

FIG. 2 is a schematic view showing the procedure of the immunoassay of the present invention wherein the analyte antigen in the sample and the non-magnetic particles immobilized with the antigen are competitively reacted. Two cases with different contents of the analyte antigen are shown to explain that such difference will result in a difference in the amount of the non-magnetic particles remaining in the reaction container.

FIG. 3 shows a calibration curve depicted by measuring absorbance of standard CRP samples of known concentrations at a wavelength of 570 nm.

FIG. 4 shows a calibration curve depicted by measuring absorbance of standard Lp(a) samples of known concentrations at a wavelength of 570 nm.

### DETAILED DESCRIPTION OF THE INVENTION

Next, the present invention is described in further detail.

The kit of the present invention for immunologically assaying an analyte biological substance in a sample by utilizing an antigen-antibody reaction comprises insoluble magnetic particles immobilized with a substance that immunologically binds to the analyte substance (hereinafter referred to as magnetic particles (A)); and insoluble non-magnetic particles having an absorption wave length of a particular range immobilized with the analyte substance (hereinafter referred to as non-magnetic particles (B)). These particles may be in the form a dispersion.

In the present invention, the samples containing the analyte substance to be assayed by utilizing the antigen-antibody reaction may typically be a human or animal body fluid. Such body fluids include whole blood, serum, plasma, urine, lymph, spinal fluid, articular fluid, saliva, perspiration, milk, gastric juice, pancreatic juice, intestinal juice, bile, and tear.

Among such body fluids, use of the present invention is advantageous for assaying colored samples such as whole blood, milk, bile and the like that previously required a pretreatment before the assay, and in particular, for assaying whole blood since only a minute amount of the sample is required in the present invention.

The analyte substance to be assayed may be either an antigen or an antibody in the sample. When the analyte substance is an antigen, the antigen must have an antibody producing ability. The substance that immunologically binds to the analyte substance may be an antigen or an antibody that specifically binds to said analyte antigen or antibody, and in the case of the antibody, it may be an anti-antibody.

Antibodies are generally classified into polyclonal antibodies and monoclonal antibodies; and into 5 classes, namely, IgA, IgM, IgG, IgE, and IgD. Since an antibody is a protein, an antibody itself has immunogenicity or antigenicity, and the antibody itself may serve as an antigen.

The antigens that may be employed in the present invention include the antibodies as described above, and in addition, Fab and F(ab')₂ fragments produced by cleaving the above-described antibodies with papain or pepsin, γ-globulin, and various other substances. More illustratively, the antigens that may be used include albumin; HCG (human chorionic gonadotropin), AFP (alpha fetoprotein), CEA (carcinoembryonic antigen), CRP(C-reactive protein), cardiolipin antigen, HBs(hepatitis B surface antigen), human growth hormone, hemoglobin, Lp(a), (lipoprotein (a)), and other proteins; Apo-AI, Apo-AII, Apo-CII, Apo-CIII, Apo-B, Apo-E, and other apolipoproteins; blood group substances that are sugar chain antigens including A antigen, B antigen, O antigen, Le^{a} antigen, Le^{b} antigen and the like; and steroid hormones, concanavalin A, various prostaglandins and other haptens that are bound to a carrier; and the like.

The antibodies that may be employed in the present invention also include anti-albumin antibody, anti-Lp(a) antibody, anti-CRP antibody, anti-human growth hormone antibody, anti-HCG antibody, anti-AFP antibody, anti-CEA antibody, anti-human coagulation factor antibody, anti-HBs antibody, and other antibodies against proteins etc.; anti-IgA antibody, anti-IgM antibody, anti-IgG antibody, anti-IgE antibody, anti-IgD antibody, anti-γ-globulin antibody and other immunoglobulin antibodies; anti-steroid hormone antibody, anti-DNA antibody, anti-prostaglandin antibody, and other antibodies against haptens.

In the present invention, non-magnetic particles are immobilized with the analyte antigen or antibody, and such non-magnetic particles or such non-magnetic particles dispersed in an aqueous solution are combined with the magnetic particles (A) as will be described below to constitute the kit for immunologically assaying the biological substance, and the thus prepared kit is used for the immunoassay.

The non-magnetic particles that can be used in the present invention may comprise an organic polymer substance or an inorganic substance.

Exemplary particles of organic polymer substances include gelatin particles, polystyrene particles, styrene-butadiene copolymer particles, and styrene-(meth)acrylate copolymer particles, and these particles may be prepared in the form of a latex by emulsion polymerization.

Exemplary monomers that can be used for preparing the (meth)acrylate copolymer particles as mentioned above include 2-hydroxyethyl (meth)acrylate, 2-hydroxypropyl (meth)acrylate, 1-methyl-2-hydroxyethyl (meth)acrylate, glycerol monomethacrylate, 2-acrylamide-2-methylpropanesulfonate, 2-sulfoethyl methacrylate, acid phosphoxyethyl methacrylate, 3-chloro-2-acid phosphoxypropyl methacrylate, acid phosphoxypropyl methacrylate, ethyl (meth)acrylate, n-butyl (meth)acrylate, i-butyl (meth)acrylate, 2-ethylhexyl (meth)acrylate, lauryl methacrylate, cyclohexyl methacrylate, (meth)acrylamide, N-methylol acrylamide, N-butoxymethylacrylamide, glycidyl (meth)acrylate, methylglycidyl (meth)acrylate, etc.

Exemplary inorganic substances include silica, alumina, and titania.

The non-magnetic particles should have a particular range of absorption wave length, and such particles are preferably prepared by addition of a colorant in a known manner. The particular absorption range of the wave length is from 190 to 1,000 nm, and absorption peak should be included within the range. The colorant added should be the one that would enable the measurement of the absorbance within such range of the wave length. Use of the colorant that would enable the measurement of absorbance at a wave length in the range of from 350 to 700 nm is particularly preferred since detection of the ultraviolet absorption by the proteins in the sample would be prevented.

It is not necessary that the non-magnetic particles are colored, and the non-magnetic particles may be opaque.

Particle size of the non-magnetic particles is not limited to any particular range, and may preferably be in the range of from 0.01 to 100 µm, and more preferably, from 0.1 to 30 µm. The non-magnetic particles having a particle size of less than 0.01 µm are likely to undergo agglutination upon immunization with the antibody, and in addition, collection by centrifugation of such particles is rather difficult. On the other hand, the non-magnetic particles having a particle size in excess of 100 µm will precipitate in an aqueous solution in a short period, and such particles should lack the floatability that allows the particles to undergo the antigen-antibody reaction.

The magnetic particles used in the present invention are those magnetized by incorporating a magnetic substance such as iron or a magnetic iron oxide in the above-described particles of the organic polymer substance or the inorganic substance, or by coating the nucleus of the above-described particles of an organic polymer substance or an inorganic substance with a ferrite to thereby produce ferrite-coated particles.

The magnetic particles may preferably have a particle size in the range of from 0.01 to 100 µm, and more preferably, from 0.1 to 30 µm. The magnetic particles of less than 0.01 µm in size are difficult to prepare, and even if prepared, such particles are likely to have insufficient susceptibility for collection by magnetism. The magnetic particles having an average particle size in excess of 100 µm will suffer from insufficient dispersibility due to their excessively large surface area, leading to reduced assay sensitivity.

In the present invention, the magnetic particles may be those prepared by further coating the magnetic particles that have been prepared as described above with a polymer.

The polymers that may be used for such coating treatment include synthetic polymer such as silane, Nylon and polystyrene; natural polymers such as gelatin, chitin, and other proteins, and natural rubber; and copolymers and mixtures thereof.

In the present invention, the non-magnetic particles are immobilized with the above-described analyte substance present in the sample, and the magnetic particles are immobilized with the substance that immunologically binds to the analyte substance, and the resulting particles are the magnetic particles (A) and the non-magnetic particles (B), respectively.

The sample specimen may contain biological substances such as erythrocytes, hemoglobin, bilirubin, and the like as well as drugs such as phenobarbital, phenytoin, digoxin, imipramine, theophylline, penicillin, and the like, and such substances in the sample specimen should interfere with the assay to adversely affect the sensitivity and the accuracy of the assay. In the present invention, magnetic particles immobilized with a substance that immunologically binds to such assay-interfering substance (hereinafter referred to as magnetic particles (C)) may be prepared and added to the assay system in addition to the magnetic particles (A) and the non-magnetic particles (B) to thereby remove the substance that may interfere with the assay. The magnetic particles used for the preparation of the magnetic particles (C) may be selected from those particles that are used for the preparation of the magnetic particles (A). It is also possible to use two or more types of the magnetic particles (C) for the assay.

In the present invention, the substance that "immunologically binds" to the analyte substance or the assay-interfering substance is the substance that "specifically binds" to such substance. When the analyte substance or the assay-interfering substance is the above-mentioned antigen, the substance that "immunologically binds" to such an antigen is an antibody against such an antigen, and when the analyte substance or the assay-interfering substance is the above-mentioned antibody, the substance that "immunologically binds" to such an antibody is an antigen or an anti-antibody against such an antibody.

The procedure of loading or immunizing the particles as described above with the analyte substance or the substance that immunologically binds to the analyte substance does not differ whether the particles are magnetic or non-magnetic, and the analyte substance or the substance that immunologically binds to the analyte substance may be physically adsorbed on the particles or chemically immobilized on the particles.

More illustratively, the physical adsorption may be carried out by reacting said antigen or antibody with said particles in an adequate buffer solution. The buffer solutions that may be used in such reaction include phosphate buffer saline, Tris-HCl buffer solution, and carbonate buffer solution. An adequate buffer solution that may fulfill an appropriate buffering action may be selected depending on the pH required for the adsorption reaction. The reaction will readily proceed when the particles are mixed with the analyte substance or the substance that immunologically binds to the analyte substance at room temperature, and the particles immobilized with the desired substance will be produced.

Chemical loading may be carried out by employing carbodiimide method or glutaraldehyde method used in the so-called peptide binding process to thereby obtain the particles immobilized with the desired substance.

The antibodies immobilized on the particles may be either polyclonal or monoclonal, and may typically be an immunoglobulin of γ-globulin, IgG, IgM or other classes, or a fragment such as F(ab')2, Fab' or the like.

The antigen immobilized on the particles may be a cell debris, a hapten bound to a carrier, a protein, an immunocomplex, a natural or synthetic high-molecular weight antigen. The carrier used for chemically binding with the hapten may be an exogenic protein such as alubumin.

The amount of the antigen or the antibody immobilized on the particles may considerably differ by such factor as the type of the particles, and in general, the adequate amount selected is in the range of from 0.001 mg/ml to 20 mg/ml, and preferably, from 0.005 mg/ml to 5 mg/ml.

The particles immobilized with the immunologically binding substance are then dispersed to 0.01 to 10% by weight in an aqueous medium such as phosphate buffer saline or Tris-HCl buffer solution that may optionally contain BSA (bovine serum albumin) or other serum, and the particles are used in the form of a latex suspension to constitute a part of the immunoassay kit of the present invention.

As described above, the immunoassay kit of the present invention may comprise at least the magnetic particles (A) immobilized with a substance that immunologically binds to the analyte substance, and one or more types of the non-magnetic particles (B) immobilized with the analyte substance.

The immunoassay kit of the present invention may further comprise one or more types of the magnetic particles (C) immobilized with a substance that immunologically binds to the substance in the sample that interferes with the assay. When such particles are in the form of a dispersion, the dispersion may have optionally added an antiseptic such as NaN₃ added thereto.

The immunoassay kit of the present invention may further comprise a standard sample used for depicting the calibration curve; an aqueous solution for diluting the standard sample; and an aqueous solution for diluting the sample. Such aqueous solution may typically be water, physiological saline, or phosphate buffer saline or Tris-HCl buffer solution as mentioned above. The aqueous medium that may contain the magnetic particles or the non-magnetic particles as described above, the standard sample for depicting the calibration curve, the buffer solution for the dilution of the standard sample, and the buffer solution for the dilution of the sample may be respectively stored in a surface treated glass container or a plastic container made from, for example, polypropylene.

The assay process of the present invention comprises the steps (1), (2) and (3) as will be described below.

When the immunoassay kit of the present invention is used, the particles (A) and (B) as described above are mixed with the sample in step (1).

With regard to the amount of the particles used, the magnetic particles (A) immobilized with the substance that immunologically binds to the analyte substance in an amount in the range of from 1/4 to 4 volumes may be used per 1 volume of the non-magnetic particles (B) immobilized with the analyte substance in view of the efficiency of the reaction between the magnetic particles (A) with the non-magnetic particles (B). More preferably, 1 to 4 volumes of the magnetic particles (A) is used per 1 volume of the non-magnetic particles (B).

The particles (A), (B) and the sample may be mixed in an arbitrary order. Preferably, the magnetic particles (A) is first mixed with the sample, and the non-magnetic particles (B) is then added to the mixture. In another preferable method the non-magnetic particles (B) is first mixed with the sample, and the magnetic particles (A) is then added to the mixture.

As mentioned above, the magnetic particles immobilized with the substance that immunologically binds to the assay-interfering substance (C) may be mixed with the magnetic particles (A) and the non-magnetic particles (B). In such case, the magnetic particles (C) may be used in an amount in the range of from 1/10 to 10 volumes, and more preferably, from 1/2 to 2 volumes per 1 volume of the non-magnetic particles (B).

The particles (A), (B) and (C) may be mixed in an arbitrary order. Preferably, the magnetic particles (C), the magnetic particles (A) and the sample are mixed together to fully promote the reactions therebetween before adding the non-magnetic particles (B) in order to attain a satisfactory assay sensitivity and reproducibility. More preferably, the magnetic particles (C) is first mixed with the sample, and the magnetic particle (A) is then added to the mixture to fully promote the reaction before adding the non-magnetic particles (B).

It is, however, also preferable to mix the particles (A), (B) and (C) and the sample all at once since such procedure would enable a simple, quick assay.

The amount of the sample added may be in the range of from 1/60 to 1/3 volume per 1 volume of the magnetic particles (A), and if desired, the sample may be diluted with a buffer or the like.

The procedure of mixing the particles with the sample is generally carried out at room temperature (20 to 30°C). However, the reaction system may be heated to 30 to 40°C to accelerate the immunological binding reaction between the substances immobilized on the particles and the analyte substance, or alternatively, cooled to 4 to 20°C to maintain stability of the reagents.

The reaction period between the particles and the analyte substance is not limited to any particular range. The reaction period of from 1 to 60 minutes, however, is preferable since the immunoreaction should be substantially completed within such period and the assay sensitivity and reproducibility would not be impaired.

Step (2) of the assay of the present invention is the step of applying magnetic field to the mixture of the particles and the sample obtained in the above-described step (1).

Intensity of the magnetic field is not limited to any particular range, and typical intensity is in the range of 1,000 to 10,000 Gauss when the total volume of the mixture is 200 µm. A commercially available magnetic gatherer such as GATHERIN manufactured by Nippon Paint Co., Ltd. may be employed.

In the step (2), the magnetic particles (A) and (C) that failed to react; the magnetic particles (A) immunologically bound to the non-magnetic particles (B); the complex formed between the magnetic particles (A) and the analyte substance; and the complex formed between the magnetic particles (C) and the assay-interfering substance are collected by means of magnetic field.

The collection by magnetism of the step (2) may be carried out for a non-limited period of time, and typically, for 1 second to 10 minutes, and preferably, for about 10 seconds to 5 minutes.

In step (3), amount of the non-magnetic particles (B) which escaped the magnetic collection is determined by measuring absorbance at a wave length in the range of from 190 to 1,000 nm, and preferably from 350 to 700 nm. When the non-magnetic particles contain the colorant as mentioned above, it would be preferable to use the wave length that is absorbed by the colorant.

The immunoassay process of the present invention is capable of qualitatively or quantitatively assaying the analyte substance in the sample.

When the analyte substance is quantitatively assayed, a calibration curve is first depicted by preparing a series of standard samples each containing the analyte substance of a known concentration and measuring the absorbance at the above-described wave length, and subsequently, the sample containing the analyte substance of unknown concentration is quantitatively assayed by measuring the absorbance to thereby determine the concentration of the analyte substance by referring the calibration curve.

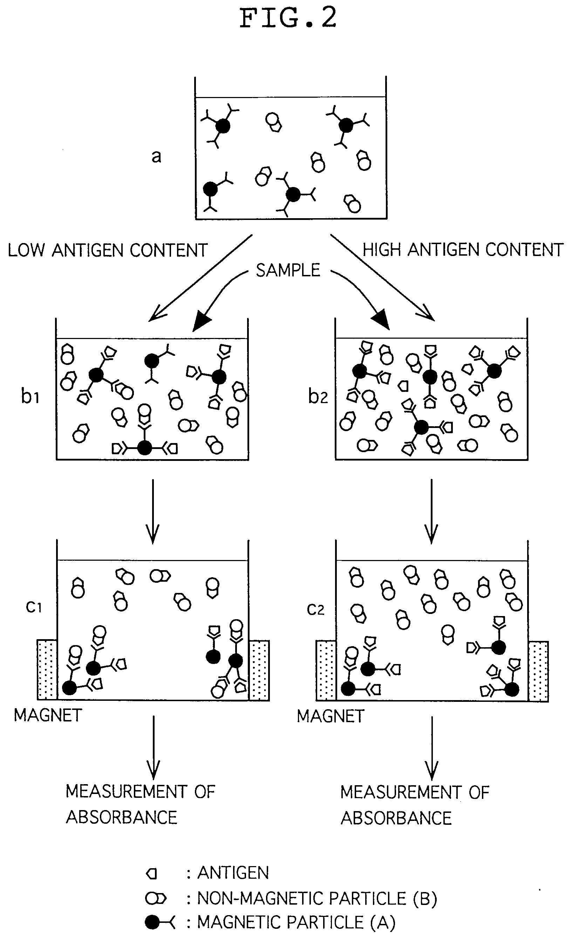

When the analyte substance is qualitatively assayed, a predetermined amount of the particles immobilized with the substance that immunologically binds to the analyte substance is added to the sample, and the magnetic components are collected by magnetism as shown in FIG. 2. The presence or the absence of the analyte substance is thereafter determined by visual inspection with naked eye.

In the present invention, the immunocomplexes formed by the competitive binding of the analyte antigen and the antigen immobilized on the non-magnetic particle (B) to the antibody on the magnetic particle (A) are collected onto the side wall of the reaction container by the magnetic field applied, and the amount of the non-magnetic particles (B) remaining in the reaction system is detected by measuring the absorbance as shown in FIG. 2, and it is not the occurrence of the agglutination that is measured in the present invention. Therefore, the present invention is free from the prozone phenomenon as described above. Since an increase in the amount of the antigen in the sample results in an increase in the amount of the immunocomplex formed between the magnetic particle (A) and the antigen in the sample, and hence, in an increase in the amount of the non-magnetic particles (B) remaining in the reaction system, the calibration curve depicted would ascend to the right.

The present invention is based on the principle that the antibody on the magnetic particle (A) should preferentially bind to the free antigen present in the reaction system before the antigen bound to the non-magnetic particles (B) which is sterically hindered by the binding to the non-magnetic particle. In other words, it is the free antigen that preferentially binds to the antibody, and the antigen of the non-magnetic particle (B) then binds to the remaining vacant antigen-binding sites, irrespective of the amount of the free antigen present in the sample.

When the amount of the antigen present in the sample is small as in the case of FIG. 2b1, amount of the complex formed by the binding of the antibody of the magnetic particle (A) and the antigen of the non-magnetic particle (B) will be increased, and consequently, the amount of the non-magnetic particle (B) remaining in the reaction container will be decreased. The absorbance measured would then be reduced (FIG. 2c1).

On the contrary, when a large amount of the antigen is present in the sample as in the case of FIG. 2b₂, amount of the complex formed by the binding of the antibody of the magnetic particle (A) and the antigen of the non-magnetic particle (B) will be reduced, and consequently, the amount of the non-magnetic particle (B) remaining in the reaction container will be increased. An increased absorbance would then be measured (FIG. 2c2).

As described above, in the present invention, positive correlation is present between the amount of the antigen present in the sample and the amount of the non-magnetic particles (B) remaining in the reaction container. In addition, the immunocomplexes formed will be collected by the magnetism irrespective of their sizes to enable the measurement of the remaining non-magnetic particles (B), and therefore, the assay is free from the prozone phenomenon associated that has with the conventional agglutination assay.

Next, measurement of CRP (C-reactive protein) in whole blood sample by the assay process of the present invention is described by way of an example. It should be noted that CRP is a β-globulin in serum whose amount increases during an inflammatory disease that involves tissue damage.

When the sample used for the assay is whole blood, the sample contains erythrocytes, hemoglobin and the like that should interfere with the assay in addition to the CRP. In such a case, magnetic particles (C) immobilized with such substance as an anti-erythrocyte antibody that specifically binds to the erythrocytes in the sample are used in admixture with the magnetic particles (A) immobilized with an anti-CRP antibody that specifically binds to the CRP in the sample and the non-magnetic particles (B) immobilized with the CRP.

When the sample used for the assay is serum or plasma, use of the magnetic particles (C) is generally not required. However, in the case where hemolysis has occurred, it would be preferred to employ magnetic particles (C) immobilized with an anti-hemoglobin antibody.

After mixing the three types of particles as described above at room temperature, the sample is mixed with the particle mixture. CRP would then compete with the CRP immobilized on the non-magnetic particles (B), and bind to the magnetic particles (A) immobilized with the anti-CRP antibody. The magnetic particles (A) immobilized with the anti-CRP antibody would preferentially bind to the free CRP in the sample to form the immunocomplex, and then to the CRP immobilized on the non-magnetic particles (B). The binding reaction is allowed to proceed for about 20 minutes.

In the meanwhile, standard sample solutions of particular concentration range are prepared from the standard CRP solution for the purpose of depicting the calibration curve, and the thus prepared standard sample solutions are mixed with various particles as in the case of the assay sample to promote the formation of the immunocomplexes.

Next, a magnetic field of the above-mentioned intensity is applied to collect the magnetic particles that failed to react and that are in the form of immunocomplexes.

In the absence of the assay-interfering substances such as erythrocytes in the sample, the components collected by the magnetism would be the immunocomplex formed between the magnetic particle (A) and the free CRP in the sample; the magnetic particle (A) that failed to react; and the immunocomplex formed between the magnetic particle (A) and the non-magnetic particle (B).

When the assay-interfering substances is present in the sample, the components collected by the magnetism would include the magnetic particle (C) that failed to react; and the immunocomplex formed between the magnetic particle (C) and the assay-interfering substance such as erythrocyte; in addition to the particles and the immunocomplexes collected in the above-described case.

In either case, the particles remaining in the reaction container escaping the collection by magnetism would be the non-magnetic CRP-immobilized particles (B).

After the collection of the free magnetic particles and the magnetic immunocomplexes as described above, the amount of the non-magnetic particles (B) remaining in the samples for calibration or the sample to be assayed is determined by measuring absorbance at an appropriate wave length in the range of from 190 to 1,000 nm.

The absorbance measurements of the calibration samples are plotted to depict the calibration curve. The CRP concentration of the assay sample is determined by comparing the absorbance measurement of the assay sample with the calibration curve and reading the CRP concentration therefrom.

The assay may be carried out in a substantially equivalent manner when CRP used in the foregoing description is substituted Lp(a), which is a protein that increases at the onset of arteriosclerotic diseases.

The present invention is further described by referring to the Examples, which by no means limit the scope of the invention.

### EXAMPLES

### Example 1

### (1) Preparation of CRP immobilized non-magnetic particles

Non-magnetic blue-colored particles having an average diameter of 0.17 µm were dispersed in 1 ml of 20mM phosphate buffer saline, pH 7.4 containing 0.02% surfactant to a solid content of 1% (w/w).

To 1 ml of the dispersion was added 100 µl of human serum (manufactured by ATAB) containing CRP at a concentration of 24.6 mg/dl, and the dispersion was stirred. The particles were immobilized by storing the dispersion in a refrigerator for 3 days.

The thus immobilized dispersion was subjected to centrifugation at 12,000 rpm for 20 minutes to remove the supernatant, and the precipitated blue colored particles were resuspended in 1 ml phosphate buffer saline containing 1% bovine serum albumin.

The procedure was repeated three times, and the particles were finally dispersed in the above-described phosphate buffer saline containing 1% bovine serum albumin to prepare a dispersion (B-1) of the CRP immobilized non-magnetic particles having a particle content of 0.2% (w/w).

### (2) Preparation of anti-CRP goat antibody immobilized magnetic particles

Magnetic particles coated with a styrene-methacrylic acid copolymer having an average particle diameter of 1.35 µm were dispersed in 1 ml of phosphate buffer saline to a solid content of 1% (w/w). To 1 ml of the dispersion was added 0.25 mg of anti-human CRP goat antibody (manufactured by ATAB), and the dispersion was stirred. The particles were immobilized by storing the dispersion in a refrigerator for 3 days.

The thus immobilized dispersion was subjected to centrifugation at 12,000 rpm for 20 minutes to remove the supernatant, and the precipitated magnetic particles were resuspended in 1 ml phosphate buffer saline containing 1% bovine serum albumin.

The procedure was repeated three times, and the particles were finally dispersed in the above-described phosphate buffer saline containing 1% bovine serum albumin. The dispersion was diluted with the above-described buffer solution to prepare a dispersion (A-1) of the anti-CRP goat antibody immobilized magnetic particles having a particle content of 0.8% (w/w).

### (3) Preparation of anti-human erythrocyte antibody immobilized magnetic particles

Magnetic particles coated with a styrene-methacrylic acid copolymer having an average particle diameter of 1.35 µm were dispersed in 1 ml of phosphate buffer saline to a solid content of 1% (w/w). To 1 ml of the dispersion was added 0.25 mg of anti-human erythrocyte rabbit antibody (manufactured by ORGNON TEKNIKA; total protein, 23.5 mg/ml), and the dispersion was stirred. The particles were immobilized by storing the dispersion in a refrigerator for 3 days.

The dispersion was subjected to centrifugation at 12,000 rpm for 20 minutes to remove the supernatant, and the thus precipitated magnetic particles were resuspended in 1 ml phosphate buffer saline containing 1% bovine serum albumin. The procedure was repeated three times, and the particles were finally dispersed in the above-described phosphate buffer saline containing 1% bovine serum albumin. The dispersion was diluted with the above-described buffer solution to prepare a dispersion (C-1) of the anti-human erythrocyte rabbit antibody immobilized magnetic particles having a particle content of 0.4% (w/w).

### (4) Preparation of kit

A CRP assay kit was constituted from the dispersions respectively containing the CRP immobilized non-magnetic particles (B-1), the anti-CRP goat antibody immobilized magnetic particles (A-1), and the anti-human erythrocyte rabbit antibody immobilized magnetic particles (C-1) prepared in the above (1) to (3); a CRP standard solution (manufactured by ATAB) for preparing a calibration curve; and a buffer solution (30 mM phosphate buffer saline) for diluting the standard solution.

### (5) Procedure

1) 50 µl of the magnetic particle dispersion (A-1), 50 µl of the magnetic particle dispersion (C-1), and 10 µl of sample blood were dispensed into wells of 96 well microplate, and the reaction was allowed to proceed for 20 minutes at room temperature.
   Samples of known concentrations were also prepared for the purpose of depicting a calibration curve by using the above-mentioned standard solution, and 10 µl of the thus prepared sample was dispensed in the well instead of the sample blood together with the dispersions (B-1) and (C-1), and the reaction was allowed to proceed for 20 minutes at room temperature.
2) 100 µl of the non-magnetic blue colored particle dispersion (B-1) was added to the reaction mixture of the above step 1) in the well, and the reaction was allowed to proceed for 20 minutes at room temperature.
3) Next, a magnetic particle-gathering apparatus (GATHERIN, manufactured by Nippon Paint Co., Ltd.) was placed underneath the microplate to gather the magnetic particles for 5 minutes, and the reaction mixture was allowed to stand at room temperature.
4) The non-magnetic blue colored particles (B-1) that escaped the magnetic gathering were evaluated for the concentration by determining the absorbance at 570 nm with a microplate reader (manufactured by TOSO).

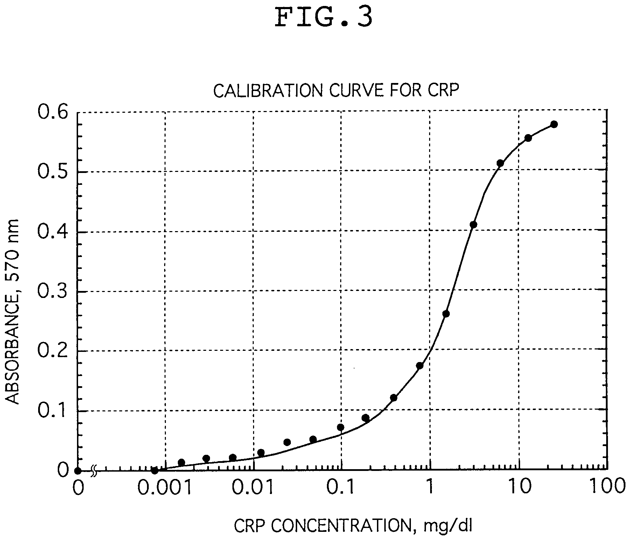

Absorbance at 570 nm was also measured for the standard samples of known concentrations prepared in the above 1) to depict a calibration curve. The resulting calibration curve is shown in FIG. 3.

As evidently shown from the concentration range of the calibration curve of FIG. 3, CRP content of the whole blood can be determined at a high sensitivity by using a minute amount of the sample blood.

### Example 2

### (1) Preparation of Lp(a) immobilized non-magnetic particles

Non-magnetic blue-colored particles having an average diameter of 0.17 µm were dispersed in 1 ml of 20mM phosphate buffer saline, pH 7.4 containing 0.02% surfactant to a solid content of 1% (w/w).

To 1 ml of the dispersion was added 0.25 mg of human Lp(a) serum (manufactured by IIC, 100 mg/dl), and the dispersion was stirred. The particles were immobilized by storing the dispersion in a refrigerator for 3 days.

The thus immobilized dispersion was subjected to centrifugation at 12,000 rpm for 20 minutes to remove the supernatant, and the thus precipitated blue colored particles were resuspended in 1 ml phosphate buffer saline containing 1% bovine serum albumin.

The procedure was repeated three times, and the particles were finally dispersed in the above-described phosphate buffer saline containing 1% bovine serum albumin.

The dispersion was diluted with the above-described buffer solution to prepare a dispersion (B-2) of the Lp(a) immobilized non-magnetic blue colored particles having a particle content of 1% (w/w).

### (2) Preparation of anti-Lp(a) goat antibody immobilized magnetic particles

Magnetic particles coated with a styrene-methacrylic acid copolymer having an average particle diameter of 1.35 µm were dispersed in 1 ml of phosphate buffer saline to a solid content of 1% (w/w). To 1 ml of the dispersion was added 0.25 mg of anti-human Lp(a) goat antibody (manufactured by International Enzymes, 20.3 mg/ml measured by Becker method), and the dispersion was stirred. The particles were immobilized by storing the dispersion in a refrigerator for 3 days.

The thus immobilized dispersion was treated with a magnetic separator to remove the liquid content in the dispersion, and the thus separated magnetic particles were resuspended in 1 ml phosphate buffer saline containing 1% bovine serum albumin. The procedure was repeated three times, and the particles were finally dispersed in the above-described phosphate buffer saline containing 1% bovine serum albumin.

The dispersion was diluted with the above-described buffer solution to prepare a dispersion (A-2) of the anti-Lp(a) goat antibody immobilized magnetic particles having a particle content of 1% (w/w).

### (3) Preparation of anti-human erythrocyte antibody immobilized magnetic particles

Magnetic particles coated with a styrene-methacrylic acid copolymer having an average particle diameter of 1.35 µm were dispersed in 1 ml of phosphate buffer saline to a solid content of 1% (w/w). To 1 ml of the dispersion was added 0.25 mg of anti-human erythrocyte rabbit antibody, and the dispersion was stirred. The particles were immobilized by storing the dispersion in a refrigerator for 3 days.

The dispersion was treated with a magnetic separator to remove the supernatant, and the separated magnetic particles were resuspended in 1 ml phosphate buffer saline containing 1% bovine serum albumin. The procedure was repeated three times, and the particles were finally dispersed in the above-described phosphate buffer saline containing 1% bovine serum albumin.

The dispersion was diluted with the above-described buffer solution to prepare a dispersion (C-2) of the anti-human erythrocyte rabbit antibody immobilized magnetic particles having a particle content of 1% (w/w).

### (4) Preparation of kit

An immunoassay kit was constituted from the dispersions respectively containing the particles (B-2), (A-2) and (C-2) prepared in the above (1) to (3); a Lp(a) standard solution for preparing a calibration curve; and a buffer solution (30 mM phosphate buffer saline) for diluting the standard solution.

### (5) Selection of wavelength for the measurement

An absorbance profile of the non-magnetic blue colored particles (B-2) was depicted by scanning the absorbance at wavelength in the range of from 190 nm in the ultraviolet range to 1,000 nm in the visible range, and 570 nm was selected for the wavelength of the measurement.

### (6) Procedure

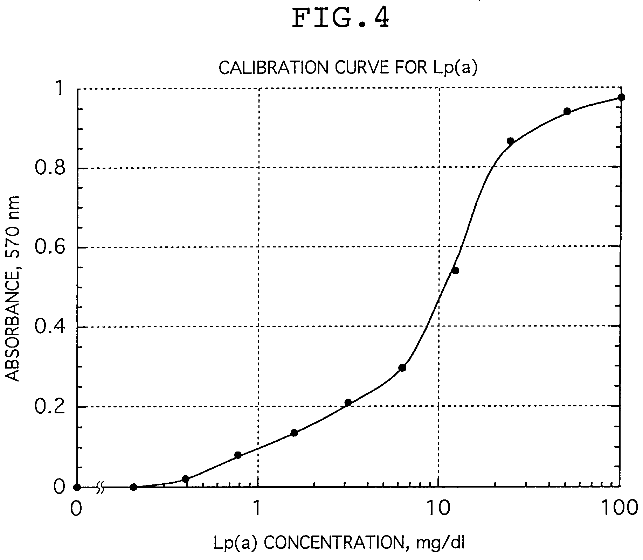

1) Samples were prepared by mixing the standard Lp(a) solution (100 mg/dl) with the blood to Lp(a) concentrations of 1, 10, 50, and 100 mg/dl.
2) 10 µl of said sample and 100 µl phosphate buffer saline containing 1% bovine serum albumin were mixed in wells of 96 well microplate.
   The standard Lp(a) solution was also mixed with the phosphate buffer saline in other wells of the 96 well microplate.
3) 60 µl of the magnetic particle dispersion (A-2) and 20 µl of the magnetic particle dispersion (C-2) were added to each well, and the reaction was allowed to proceed for 10 minutes at room temperature.
4) 20 µl of the non-magnetic blue colored particles dispersion (B-2) was added to each well, and the reaction was allowed to proceed for 10 minutes at room temperature.
5) Next, a magnetic particle-gathering apparatus, GATHERIN was placed underneath the microplate, and the apparatus was turned on for 5 minutes to gather the immunocomplex formed by the Lp(a) antigen of the non-magnetic blue colored particle (B-2) and the antibody of the magnetic particle (A-2), the immunocomplex of the erythrocyte and the anti-human erythrocyte antibody of the magnetic particle (C); and the magnetic particles that failed to undergo the reaction on the side wall of the well.
6) The non-magnetic blue colored particles (B-2) remaining in the reaction mixture in the well were evaluated for their concentration by determining the absorbance at 570 nm with a microplate reader.
   A calibration curve shown in FIG. 4 was also depicted for the concentration in the range of from 0 to 100 mg/dl.
7) Measurements of the whole blood samples were read by referring the above-mentioned calibration curve. The values actually measured for the preparation of the Lp(a) calibration curve and for the whole blood samples are shown in Table 1 (n=5).

**Table 1**

| | | | | |
|---|---|---|---|---|
| Lp(a) concentration in whole blood sample, mg/dl | 1 | 10 | 50 | 100 |
| Absorbance measured | 0.09 | 0.51 | 0.94 | 0.99 |
| Lp(a) concentration measured, mg/dl | 1.1 | 11.3 | 50.0 | 100 |

As shown in FIG. 4, the calibration depicted for the Lp(a) ascends to the right as in the case of the CRP, and no prozone phenomenon was observed. A measurement at a high sensitivity is thereby enabled.

Furthermore, Lp(a) in whole blood could be quantitatively measured over a wide range from a low concentration to a high concentration without any pretreatment such as serum separation.

As shown in the above-described Examples, the immunoassay process of the present invention has enabled a convenient, quick assay at a high sensitivity without necessitating B/F (bound/free) separation as in the case of enzyme immunoassay or radioimmunoassay.

### Merits of the Invention

According to the present invention, the immunocomplex formed between the analyte substance in the sample and the magnetic particles immobilized with the substance that would immunologically bind to the analyte substance may be removed by means of magnetism, and therefore, measurement of the amount of the remaining non-magnetic particles would provide a calibration curve that ascends to the right with no prozone phenomenon. A immunoassay kit and an immunological process using such kit that meet the commercial needs are thereby provided.

The calibration curve obtained in the immunoassay process of the present invention is depicted not by detecting the presence of the agglutination but on the bases of the measurements of the amount of the non-magnetic particles remaining in the reaction solution after the collection of the magnetic particles by magnetism. Therefore, the calibration curve depicted in the present invention does not exhibit prozone phenomenon as seen in the agglutination process. Furthermore, in the present assay process, the amount of the remaining non-magnetic particles is directly measured by means of absorbance, and no troublesome procedures such as B/F separation and the subsequent washing as required in the case of enzyme immunoassay or radioimmunoassay are required. Accordingly, use of the immunoassay kit of the present invention should enable convenient, quick determination of the concentration of the target analyte substance.

In addition, the immunoassay process of the present invention requires a quite minute amount of the sample for the assay, and measurement at a high sensitivity is possible even when the sample is whole blood. Therefore, the immunoassay process of the present invention is particularly useful in such case as a new born baby wherein only a small amount of blood can be collected.

## Claims

1. A kit for immunologically assaying an analyte biological substance in a sample by utilizing an antigen-antibody reaction comprising
insoluble magnetic particles immobilized with a substance that immunologically binds to the analyte substance, and
insoluble non-magnetic particles having an absorption wave length of a particular range immobilized with the analyte substance.

2. A kit for immunologically assaying an analyte biological substance according to claim 1 further comprising
insoluble magnetic particles immobilized with a substance that immunologically binds to a substance in the sample that interferes with the assay.

3. A process for assaying an analyte biological substance in a sample by utilizing an antigen-antibody reaction comprising the steps of
(1) mixing a dispersion of insoluble magnetic particles immobilized with a substance that immunologically binds to the analyte substance and a dispersion of insoluble non-magnetic particles having an absorption wave length of a particular range immobilized with the analyte substance with the sample in an arbitrary order to produce a mixture;
(2) applying a magnetic field to said mixture to remove complexes of said magnetic particle and said analyte substance formed in said mixture together with the magnetic particles that failed to bind immunologically; and
(3) determining concentration of said non-magnetic particles remaining in the mixture by measuring their absorbance.

4. A process for assaying an analyte biological substance in a sample according to claim 3 wherein
insoluble magnetic particles immobilized with a substance that immunologically binds to a substance in the sample that interferes with the assay are additionally added to said mixture; and
complex of said magnetic particle and said assay interfering substance formed in said mixture is additionally removed by the magnetic field.
